# EUROPEAN PATENT SPECIFICATION

(11) **EP 2 438 923 B1**
(45) Date of publication and mention of the grant of the patent: **18.03.2015**
(21) Application number: 09845576.9
(22) Date of filing: 26.06.2009
(51) Int. Cl.: A61K 36/185, A61P 3/04, A61K 31/341

(54) **COMPOSITION FOR PREVENTING OR TREATING OBESITY-RELATED DISEASES MEDIATED BY THE ACTIVATION OF AMPK AND INCLUDING 2,5-BIS-ARYL-3,4-DIMETHYLTETRAHYDROFURAN LIGNANS AS ACTIVE INGREDIENTS**
ZUSAMMENSETZUNG ZUR PRÄVENTION ODER BEHANDLUNG VON FETTLEIBIGKEITSBEDINGTEN ERKRANKUNGEN DURCH DIE AKTIVIERUNG VON AMPK UND MIT 2,5-BIS-ARYL-3,4-DIMETHYLTETRAHYDROFURANLIGNANEN ALS WIRKSTOFFE
COMPOSITION, POUR LA PRÉVENTION OU LE TRAITEMENT D'AFFECTIONS LIÉES À L'OBÉSITÉ ET MÉDIÉES PAR L'ACTIVATION D'AMPK, ET DONT LES PRINCIPES ACTIFS DONT DES LIGNANES DE 2,5-BIS-ARYL-3,4-DIMÉTHYLTÉTRAHYDROFURANE

(30) Priority: 01.06.2009 KR 20090048065
(43) Date of publication of application: 11.04.2012
(73) Proprietor: Industry-academic Cooperation Foundation, Chosun University, Gwangju 501-759 (KR); Amorepacific Corporation, Seoul 140-777 (KR)
(72) Inventor: OH, Won Keun, Gwangju 501-759 (KR); NGUYEN, Phi Hung, Quang Ninh province 36000 (VN); LE, Thi Van Thu, Hanoi 10000 (VN); KANG, Hu Won, Jeonnam 520-240 (KR); SHIN, Eui Seok, Yongin-si Gyeonggi-do 446-570 (KR); CHIO, Jin Kyu, Suwon-si Gyeonggi-do 443-370 (KR); SEO, Dae Bang, Yongin-si Gyeonggi-do 446-760 (KR); LEE, Sang Jun, Seongnam-si Gyeonggi 463-010 (KR)
(74) Representative: Hasler, Erich
(86) International application number: PCT/KR2009/003471
(87) International publication number: WO 2010/140734

(56) References cited:
- WO-A1-2006/073285
- WO-A2-2007/001150
- WO-A2-2008/156331
- KR-A- 20070 107 853
- KR-A- 20090 039 650
- DATABASE WPI Week 199748 Thomson Scientific, London, GB; AN 1997-516045 XP002685217, -& HU 208 391 A (VICZIAN G) 28 March 1996 (1996-03-28)
- JUN YOUNG CHO ET AL: "Isolation and antifungal activity of lignans fromMyristica fragrans against various plant pathogenic fungi", PEST MANAGEMENT SCIENCE, vol. 63, no. 9, 20 July 2007 (2007-07-20), pages 935-940, XP55041019, ISSN: 1526-498X, DOI: 10.1002/ps.1420
- SU-UI LEE ET AL: "Machilin A Isolated from Myristica fragrans Stimulates Osteoblast Differentiation", PLANTA MEDICA, vol. 75, no. 02, 1 February 2009 (2009-02-01), pages 152-157, XP55041021, ISSN: 0032-0943, DOI: 10.1055/s-0028-1112197
- SONG, MYOUNG CHONG ET AL: 'Phenylpropanoids from Myristica fragrans Houtt' J. KOREAN SOC. APPL. BIOL. CHEM. vol. 47, no. 3, 2004, pages 366 - 369, XP008151277
- ALPANA RAM ET AL.: 'Hypolipidaemic effect of Myristica fragrans fruit extract in rabbits' JOURNAL OF ETHNOPHARMACOLOGY vol. 55, 1996, pages 49 - 53, XP008150579
- SENUGMI YANG ET AL.: 'Inhibition of protein tyrosine phosphatase 1B by lignans from Myristica fragrans' PHYTOTHERAPY RESEARCH vol. 20, 2006, pages 680 - 682, XP008150801
- KIM, KAP-JOON ET AL.: 'Lignans from Myristica fragrans' YAKHAK HOEJI vol. 46, no. 2, 2002, pages 98 - 101, XP009112740
- KIM, SOO-HWAN ET AL.: 'Isolation and quantitative determination method validation of myristicin from Myristica fragrans Houttuyn' KOR. J. PHARMACOGN. vol. 38, no. 1, 2007, pages 19 - 21, XP008150818

## Description

### BACKGROUND

### 1. Field

The present disclosure relates to a lignan compound which activates AMP-activated protein kinase (AMPK) derived from nutmeg (*Myristica fragrans*) and a composition including the same as an active ingredient. More particularly, the present disclosure relates to a 2,5-bis-aryl-3,4-dimethyltetrahydrofuran lignan compound produced by extracting nutmeg with an aqueous solution of 10-30% ethanol, which activates AMPK and is effective in preventing and treating metabolic syndrome such as obesity, diabetes, hyperlipidemia and cardiovascular diseases, and a composition for preventing and treating diseases mediated by the activation of AMPK comprising the same as an active ingredient.

### 2. Description of the Related Art

With the improvement in dietary lives thanks to the recent economic development and improvement of living standards, calorie intake is increasing rapidly. However, insufficient calorie consumption through exercise results in increase of obesity. Obesity is not only unattractive for young people who prefer slim bodies but it also is reported to, when continued, cause various adult diseases such as hypertension, diabetes, hyperlipidemia, coronary artery disease, etc. as well as breast cancer, uterine cancer, colon cancer, and is now considered as a severe disease (J. Biol. Chem., 273, 32487-32490 (1998); Nature, 404, 652-660 (2000)).

Currently available obesity-treating drugs can be largely classified into those that affect appetite by acting on the central nervous system and those that inhibit absorption by acting on the gastrointestinal tract. The drugs that act on the central nervous system include those inhibiting the serotonin (5-HT) nervous system such as fenfluramine, dexfenfluramine, etc., those acting on the noradrenaline nervous system such as ephedrine, caffeine, etc., and those inhibiting obesity by acting on both the serotonin and noradrenaline systems such as sibutramine, etc. In addtion, as drugs that inhibit obesity by acting on the gastrointestinal tract, orlistat, which reduces fat absorption by inhibiting lipase in the tract and approved for treatment of obesity, is a typical example. However, among them, fenfluramine, etc were withdrawn from the market because of side effects such as pulmonary hypertension or heart valve disease, and other drugs are inapplicable to patients with heart failure or renal diseases because of such problems as reduced blood pressure, lactic acidosis, etc.

Accordingly, the inventors were intrigued by the regulatory mechanism of energy metabolism in order to find an improved method for the treatment of obesity. Since the preventive and therapeutic agents for metabolic syndrome are taken for a long period of time and the drug targets are mitochondria involved in the energy regulation, the drugs should have better safety (i.e., lower toxicity) than those targeting other targets. Thus, the inventors have explored an agent activating the enzyme AMP-activated protein kinase (AMPK) from natural products.

AMPK is a kinase whose activity is regulated in response to the cellular energy state (AMP/ATP ratio) such as nutrition, exercise, stress, or the like. The enzyme is known to affect various physiological processes by regulating the phosphorylation of enzymes involved in cellular energy metabolism, including glucose transport, fatty acid synthesis, cholesterol synthesis, or the like (Annu. Rev. Pharmacol. Toxicol., 47, 185-210, 2007).

According to recent reports, AMPK is involved in glucose uptake in muscle cells, stimulated by exercise and AMPK is known to play a role of a sensor monitoring cellular energy potential. During exercise or in starved state, muscle cells, hepatocytes and adipocytes stop synthesizing fats and glycogens to supply the necessary energy, and provide the energy required by the body by degrading stored fats. Also, this enzyme is known as an intracellular signal transducer of leptin and adiponectin secreted from adipocytes. Especially, adiponectin, which is observed in low levels in obese people, is considered as highly associated with obesity-induced insulin resistance and, thus, AMPK activators are emerging as promising drug targets of obesity (Nat. Rev. Mol. Cell. Biol., 8(10), 774-785, 2007).

Metformin has been used as an oral antidiabetic drug without any knowledge about its mechanism. As it is known that the drug activates AMPK, many groups have developed drugs targeting AMPK. Australia's Garban et al. reported in 2008 that four ingredients extracted from bitter melon activate the enzyme AMPK which is well known to be involved in regulation of metabolism in the body (Chem. Biol., 15(5), 263-273, 2007).

In general, among the methods of developing new drugs, to explore new active ingredients from the natural medicines used in the traditional medicine is more likely to be successful than to experimentally modify the existing drugs. Since the active ingredients have been used for a long time, the resulting drug is relatively safe from the toxicity problem.

Nutmeg tree (*Myristica fragrans*) is an evergreen tree belonging to the family *Myristicaceae,* indigenous to Sumatra and Java. It is a dioecious plant growing about 10-20 m tall. The fruit is oval-shaped with a seed enclosed by reddish covering, and the seed is called nutmeg. Nutmeg has been long been used as flavoring in food such as sauce. In the oriental medicine, it has been used as an aromatic stomachic to treat diarrhea, abdominal distension, vomiting, loss of appetite, or the like.

The inventors of the present disclosure have isolated 2,5-bis-aryl-3,4-dimethyltetrahydrofuran lignan compounds from nutmeg and found out that these compounds activated the enzyme AMPK and show activity when administered to an animal. Thus, it was found out that the compound of the present disclosure can be used to metabolic syndromes including obesity.

However, it is reported that nutmeg induces acute toxicity when administered in a high dose. The nutmeg extract contains the toxic alkylbenzene derivatives including myristicin, elemicin and safrole. In the human body, they are known to be converted into amphetamine derivatives and exhibit toxic activities similarly to psychotropic drugs. Traditionally, nutmeg has been immersed in limewater for about a day and dried by heating in order to relieve the toxicity.

The most abundant toxic substance in nutmeg is myristicin (Yagaku Zasshi, 128(1), 129-133, 2008). It is reported that the methanol extract of nutmeg contains 2.1 % of myristicin on average (Natural Toxins, 5, 186-192, 1997; Korean Journal of Pharmacognosy, 38(1), 19-21, 2007).

The inventors of the present disclosure have established an extraction condition under which the active ingredients are extracted at maximum concentrations from nutmeg with minimized concentration of myristicin for the development of a composition for preventing and treating metabolic syndrome including obesity.

The preceding patents relating to fractions of nutmeg-related extracts include "Composition for inhibiting cholesterol esterase comprising galenical extract (Korean Patent Registration No. 399529)", "Pharmaceutical composition for preventing or treating diabetes mellitus containing extract of oriental herb as active ingredient (Korean Patent Registration No. 795976)" and "Pharmaceutical composition for preventing or treating coronary heart disease or arteriosclerosis containing extract of oriental herb as active ingredient (Korean Patent Registration No. 793204)". However, these patents use the nutmeg extract itself without elucidating the active ingredients and are not closely related to the present disclosure which is based on the active compounds included in the nutmeg extract.

The preceding patents relating to the active compounds included in the nutmeg extract include "Suppressant of toxicity induced by anticancer agent and anticancer composition containing the same (Korean Patent Registration No. 646574)", "Composition containing lignan compounds as active ingredients for treating or preventing acne (Korean Patent Registration No. 567431)", "Pharmaceutical composition for liver protection and for treating liver disease (Korean Patent Registration No. 619498)", "Pharmaceutical composition for treating or preventing inflammatory diseases comprising lignan compounds (Korean Patent Registration No. 579752)", "Pharmaceutical composition for treating or preventing type 2 diabetes (Korean Patent Registration No. 627643)", "Composition for preventing or treating PPARα-mediated disease comprising macelignan or pharmaceutically acceptable salt thereof as active ingredient (Korean Patent Registration No. 830192)" and "Pharmaceutical composition for treating or preventing neurological brain disease comprising lignan compounds (Korean Patent Registration No. 679306)". These patents disclose the various activities of macelignan which is different from the active compound of the present disclosure, 2,5-bis-aryl-3,4-dimethyltetrahydrofuran lignan, in structure and characteristics.

WO2008156331 teaches the use of austrobailignan-7 which Is a , 2,5-blsaryltetrahydrofuran lignin compound obtained by extraction from the aril of nutmeg using ethanol for the treatment of syndrome X which Is a synonym for metabolic syndrome. The process of extracting austroballignan-7 includes adding dried and ground aril of nutmeg to 75% methanol and leaving it stand at room temperature for 2 days, filtering the extracted solution and concentrating in a vacuum to obtain a methanol extract of the aril of nutmeg. The extracted solution is then fractionated with ethylacetate, butanol and water (Korean application No. 2008-112090 described in the following belongs to the same patent family).

Although Korean Patent Publication No. 2008-112090 presents austobailignan 7 as an active therapeutic agent for diabetes or PPARγ-mediated disease, the compound was not found under the extraction condition of the present disclosure. The mechanism of drug action disclosed in Korean Patent Publication No. 2008-112090 is based on the activation of PPARγ and identical to that of the glitazone-based drugs used as oral antidiabetic drug. However, the drugs are known to have the side effect of inducing obesity by increasing the number of adipocytes during the treatment.

In contrast, the 2,5-bisaryltetrahydrofuran lignan compound of the present disclosure is based on the mechanism of activating AMPK and promoting energy metabolism for prevention and treatment of obesity and metabolic syndrome, differently from the activation of PPARγ, and is free from the side effect related with PPARγ activation.

The inventors compared the activity of nectandrin B, one of the 2,5-bis-aryl-3,4-dimethyltetrahydrofuran lignan compounds presented by the present disclosure, with that of macelignan disclosed in Korean Patent Registration No. 830192, which is the most relevant to the present disclosure among the nutmeg-related patents, after orally administering macelignan or nectandrin B to animal at the same concentration, for the same period. As a result, they identified that nectandrin B has far better activity.

Differently from the preceding patents, in order to maximally extract the active substance 2,5-bis-aryl-3,4-dimethyltetrahydrofuran lignan from nutmeg while minimizing the extraction of the toxic substances myristicin and elemicin, the present disclosure provides a novel nutmeg extract composition for prevention and treatment of metabolic syndrome including obesity using an aqueous solution of 10-30% ethanol as extraction solvent. Also, the inventors of the present disclosure have further removed the trace amount of myristicin included in the nutmeg extract using an ion-exchange resin.

The inventors have extracted 2,5-bis-aryl-3,4-dimethyltetrahydrofuran lignan compounds from a 10-30% ethanol extract of nutmeg and investigated their activity using C2C12 cell lines by measuring the activated AMPKs p-AMPK and p-ACCs (ACC1 and ACC2). As a representative compound, they tested nectandrin B for a drug development model for obesity and metabolic syndrome and confirmed the activity of the compound.

### SUMMARY

The present disclosure is directed to providing a composition effective for the prevention and treatment of obesity or metabolic syndrome containing a nutmeg extract including at least one 2,5-bisaryltetrahydrofuran lignan compound having AMP-activated protein kinase (AMPK)-activating activity with the toxic substance myristicin minimized, as defined in claim 1.

The present disclosure is also directed to providing a composition effective for the prevention and treatment of obesity or metabolic syndrome containing one or more 2,5-bisaryltetrahydrofuran lignan compounds having AMPK-activating activity isolated and purified from nutmeg as active ingredient, as defined in claim 1.

### BRIEF DESCRIPTION OF THE DRAWINGS

The above and other objects, features and advantages of the present disclosure will become apparent from the following description of certain exemplary embodiments given in conjunction with the accompanying drawings, in which:
FIG. 1 shows HPLC analysis spectrum of the respective compounds obtained from the active fractions of a nutmeg extract obtained by extracting with 30% ethanol and adsorbing onto Diaion HP-20;
FIG. 2 shows HPLC analysis spectrum of a nutmeg extract extracted with 30% ethanol;
FIG. 3 shows HPLC analysis spectrum of a nutmeg extract extracted with 75% ethanol;
FIG. 4 shows HPLC analysis spectrum of a nutmeg extract extracted with 75% methanol;
FIG. 5 shows HPLC analysis spectrum obtained by adsorbing a nutmeg 30% ethanol extract onto Diaion HP-20 and eluting with 80% ethanol;
FIG. 6 shows HPLC analysis spectrum obtained by adsorbing a nutmeg 30% ethanol extract onto Diaion HP-20 and eluting with 90% ethanol;
FIG. 7 shows a result of measuring p-AMPK and p-ACC for determination of AMPK activity after treating differentiated C2C12 cell lines with 2,5-bis-aryl-3,4-dimethyltetrahydrofuran lignan compounds isolated from nutmeg at final concentration of 10 µg/mL; and
FIG. 8 compares body weight increase in a high fat diet-induced obese model after oral administration of nectandrin B, macelignan as control, or nutmeg 30% ethanol extract for 8 weeks.

### DETAILED DESCRIPTION OF EMBODIMENTS

The inventors of the present disclosure have tested various wild plants and medicinal herbs for AMP-activated protein kinase (AMPK)-activating activity and selected nutmeg as a candidate plant.

They have established an extraction condition under which the active ingredient 2,5-bisaryltetrahydrofuran lignan compounds are extracted at high concentrations from nutmeg with minimized concentration of the toxic substance myristicin, and identified that the nutmeg extract and the 2,5-bisaryltetrahydrofuran lignan compounds extracted under the extraction condition have high AMPK-activating activity and suppress the inducement of obesity.

Also, they have found out that a composition comprising a nutmeg extract extracted with an ethanol aqueous solution comprising at least one 2,5-bisaryltetrahydrofuran lignan compound (see the chemical formula 1) is useful for the prevention or treatment of obesity or metabolic syndrome via activation of AMPK.

The compound is disclosed to be useful as an agent for treatment of obesity or diabetes, an agent for prevention of obesity or diabetes, or an agent for activation of AMPK.

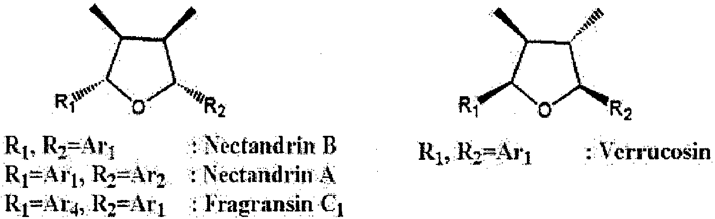

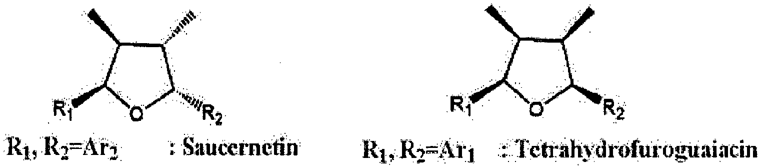

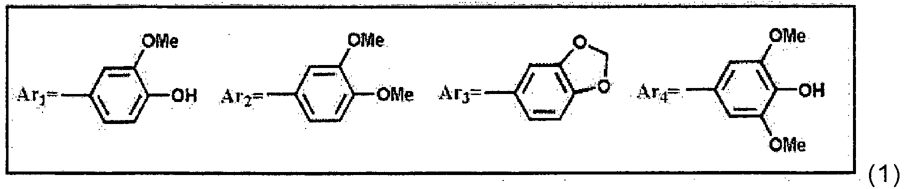

The AMPK-activator, 2,5-bisaryltetrahydrofuran lignan compound included in nutmeg may be obtained by: grinding nutmeg and extracting with an aqueous solution of ethanol; separating and purifying AMPK-activating 2,5-bisaryltetrahydrofuran lignan compounds by chromatography; investigating chemical structures as well as physical and chemical properties of the obtained 2,5-bisaryltetrahydrofuran lignan compounds; analyzing the compounds by high-performance liquid chromatography (HPLC); investigating the AMPK-activating activity of the compounds; and performing animal tests of orally administering fractions of the compounds.

A trace amount of myristicin included in the ethanol extract of nutmeg is further removed using an ion-exchange resin and the 2,5-bisaryltetrahydrofuran lignan compounds are concentrated. More specifically, the ion-exchange resin for removing myristicin may be an aromatic-based unsubstituted synthetic adsorbent resin such as Diaion HP-20, SP825, AXT204, XAD1600T or MN200 (Mitsubishi Chemical).

The inventors of the present disclosure have identified from the analysis of physical and chemical properties and NMR spectra that the compounds according to the present disclosure are nectandrin B, nectandrin A, fragransin C1, verrucosin, saucernetin and tetrahydrofuroguaiacin, and have elucidated AMPK-activating activity in animals by orally administering the compounds or fractions comprising the same.

The AMPK-activating compound according to the present disclosure can be easily obtained from nutmeg by extraction using an organic solvent (e.g., alcohol, ether, acetone, etc.), fractionation using hexane and water, column chromatography, known methods used for extraction of plant components, or a combination thereof. If necessary, the crude extract may be further purified according to commonly employed methods.

The chromatography employed in the present disclosure may be silica gel column chromatography, LH-20 column chromatography, ion-exchange resin chromatography, thin layer chromatography (TLC), high-performance liquid chromatography, or the like.

Since the 2,5-bisaryltetrahydrofuran lignan compound according to the present disclosure activates AMPK, it is effective for preventing and treating obesity or diabetes. With good bioavailability, the compound can be used advantageously. Since the 2,5-bis-aryl-3,4-dimethyltetrahydrofuran lignan compound can be easily isolated from nutmeg and has good stability, it can also be used as additive for food or medicine.

The pharmaceutical composition comprising the nutmeg extract according to the present disclosure may be prepared into oral formulations such as powder, granule, tablet, capsule, suspension, emulsion, syrup, aerosol, etc. or into parenteral formulations such as suppository or sterile injectable solution, according to commonly employed methods. The composition comprising the extract may include a carrier, excipient or diluent such as lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate or mineral oil. When formulating, a diluent or excipient such as filler, extender, binder, wetting agent, disintegrant, surfactant, etc. may be used. Solid preparations for oral administration include tablet, pill, powder, granule, capsule, etc. and are prepared by mixing the extract with one or more excipient, for example, starch, calcium carbonate, sucrose or lactose, gelatin, etc. Also, in addition to simple excipients, lubricants such as magnesium stearate or talc may be used. Liquid formulations for oral administration include suspension, internal solution, emulsion, syrup, etc. In addition to simple diluents such as water and liquid paraffin, various excipients, e.g., wetting agent, sweetener, aromatic, preservative, etc., may be included. Formulations for parenteral administration include sterilized aqueous solution, non-aqueous solution, suspension, emulsion, lyophilization preparation, or suppository. Propylene glycol, polyethylene glycol, vegetable oil such as olive oil, injectable ester such as ethyl oleate, etc. may be used as the non-aqueous solution or suspension. Witepsol, macrogol, Tween 61, cocoa butter, laurin butter, glycerogelatin, etc. may be used as a base of the suppository.

The administration dosage of the active ingredient will be different depending on age, sex, body weight of a subject, particular disease or physiological condition to be treated, severity of the disease or physiological condition, route of administration, and determination by a diagnoser. Determination of the administration dosage based on these factors is within the level of those skilled in the art. The compound of the present disclosure may be administered with a dosage of 0.01-2000 mg/kg/day, specifically 1-500 mg/kg/day. The administration may be made once or several times a day. However, the aforesaid dosage does not limit the scope of the present disclosure by any means. The extract of the present disclosure may be administered to mammals including mouse, domesticated animal and human via various routes. All possible routes of administration may be expected, including, for example, oral, rectal, intravenous, intramuscular, subcutaneous, intrauterine or intracerebroventricular routes. Since the extract of the present disclosure has little toxicity and few side effects, it may be safely administered for a long time for prophylactic purposes.

The present disclosure also provides a health functional food for prevention of obesity comprising the nutmeg extract and a sitologically acceptable food additive. The health functional food of the present disclosure may be in the form of tablet, capsule, pill, liquid, etc. The compound of the present disclosure may be added, for example, to various foods, drinks, gums, teas, vitamin complexes, functional health foods, or the like. More specifically, the present disclosure provides a health functional food for prevention and treatment of obesity or metabolic syndrome comprising the nutmeg extract as active ingredient and a sitologically acceptable food additive.

### EXAMPLES

The examples and experiments will now be described. The following examples and experiments are for illustrative purposes only and not intended to limit the scope of this disclosure. On the contrary, the examples are provided such that the present disclosure is more thorough and complete and fully understood by those skilled in the art.

### <Example 1: Preparation of ethanol extract with low myristicin content and high nectandrin B content from nutmeg>

Pulverized nutmeg (100 g) was dissolved in each solvent (500 mL, see Table 1) and active substances were extracted 3 times for 2 hours using an ultrasonic extractor. The isolated active ingredients of the extracts were used at the same concentrations. Myristicin was purchased from Sigma (Cat. No. M9237). The nutmeg extract extracted using each solvent and myristicin were analyzed by HPLC (Optima Pak C₁₈ column 4.6 x 250 mm, particle size 5 µm, flow rate 1 mL/min, UV detection: 260 nm) using MeOH/H₂O (0-32 min: 63% MeOH, 32-37 min: 63 → 100% MeOH). Table 1 shows the contents of myristicin and nectandrin B in the nutmeg extracts extracted using different solvents.

**Table 1**

| Extraction solvent | Contents | |
|---|---|---|
| | Myristicin | Nectandrin B |
| Water | 0.19% | 0.62% |
| 10% ethanol aqueous solution | 0.31% | 1.24% |
| 20% ethanol aqueous solution | 0.33% | 2.98% |
| 30% ethanol aqueous solution | 0.45% | 7.48% |
| 40% ethanol aqueous solution | 1.34% | 2.79% |
| 50% ethanol aqueous solution | 1.48% | 2.77% |
| 75% ethanol aqueous solution | 1.27% | 2.95% |
| 75% methanol aqueous solution | 1.85% | 7.47% |

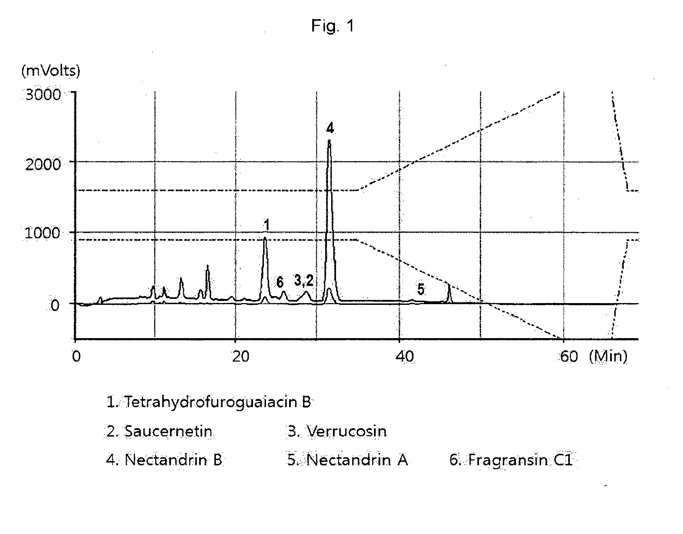

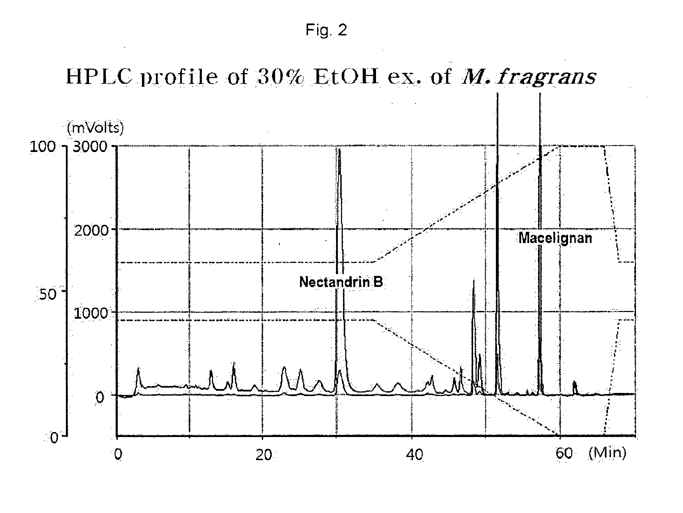

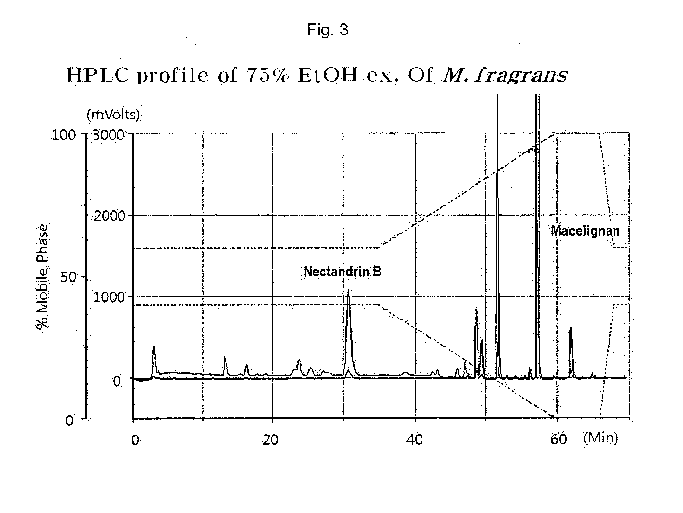

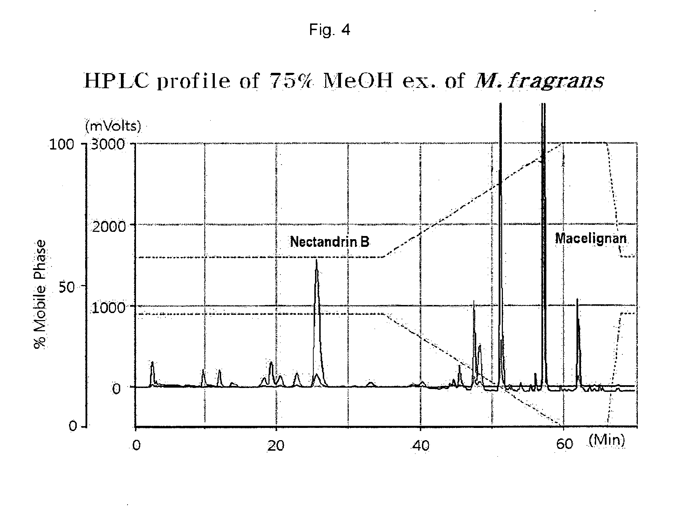

As seen from Table 1, the 30% ethanol extract had an average myristicin of 0.45%, about 3 times less than 1.27% of the 75% ethanol extract and 1.85% of the 75% methanol extract under the same condition. And, as a result of HPLC (Optima Pak C₁₈ column 4.6 x 250 mm, particle size 5 µm, flow rate 1 mL/min, UV detection: 205 and 280 nm) analysis using MeOH/H₂O (0-35 min: 60% MeOH, 35-60 min: 60 → 100% MeOH), the content of nectandrin B was highest among the 2,5-bis-aryl-3,4-dimethyltetrahydrofuran lignan compounds (see FIG. 1). The amount of the 2,5-bis-aryl-3,4-dimethyltetrahydrofuran lignan compounds in the nutmeg extract was compared using nectandrin B as reference. The 30% or less ethanol extracts had higher nectandrin B contents, as shown in FIGS. 2-4.

Accordingly, in view of maximizing the content of the active substances while minimizing the content of the toxic substance, it is the most effective to use an aqueous solution of 30% or less ethanol for extraction of the active substances from nutmeg.

### <Example 2: Further removal of myristicin from nutmeg ethanol extract>

Nutmeg (500 g) was extracted with an aqueous solution of 30% ethanol (1,000 mL) and adsorbed onto the ion-exchange resin Diaion HP-20 (500 g) by passing therethrough. Then, the extract was eluted by using 1 L of 50% ethanol, 60% ethanol, 70% ethanol, 80% ethanol, 90% ethanol, 100% ethanol and 100% acetone, respectively. Table 2 shows the degree of elution of nectandrin B and myristicin adsorbed on the Diaion HP-20 with the ethanol solution and acetone.

**Table 2**

| Eluent | Contents in eluate | |
|---|---|---|
| | Myristicin | Nectandrin B |
| 30% ethanol aqueous solution | 0.04% | 0.01% |
| 50% ethanol aqueous solution | 0.04% | 0.1% |
| 70% ethanol aqueous solution | 0.04% | 24.2% |
| 80% ethanol aqueous solution | 0.04% | 61.0% |
| 90% ethanol aqueous solution | 0.66% | 13.9% |
| 100% ethanol | 20.77% | 1.0% |
| 100% acetone | 13.71% | 3.0% |

As seen from Table 2, myristicin was hardly detected when the substances adsorbed on Diaion HP-20 were eluted using aqueous solutions of 80% or less ethanol.

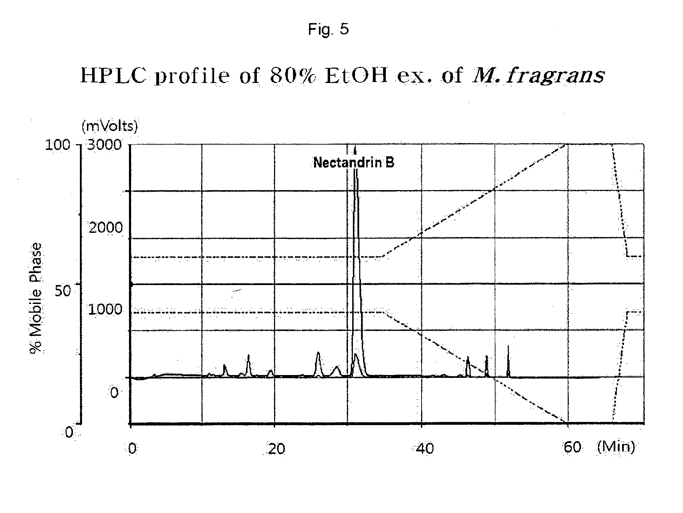

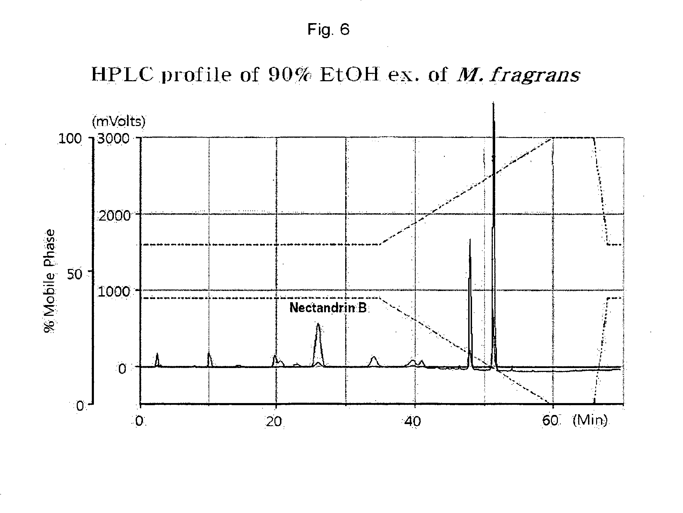

The elution of nectandrin B as one of the 2,5-bis-aryl-3,4-dimethyltetrahydrofuran lignan compounds was also monitored for the same solvents. As seen from Table 2, FIG. 5 and FIG. 6, nectandrin B was eluted with the highest concentration when 80% ethanol was used as the eluent.

### <Example 3: Identification of compounds extracted from nutmeg>

The 2,5-bis-aryl-3,4-dimethyltetrahydrofuran lignan compounds isolated from the 30% ethanol nutmeg extract in Example 1 by HPLC were analyzed by ¹H- and ¹³C-NMR. The physical and chemical properties of the 2,5-bis-aryl-3,4-dimethyltetrahydrofuran lignan compounds (Compounds 1-6) extracted from nutmeg according to the present disclosure are as follows.

### 3-1. Tetrahydrofuroguaiacin (Compound 1)

Colorless powder; ¹H-NMR: ppm (500 MHz, CDCl₃): δ 0.61 (6H, d, *J* = 6.0 Hz, 3- and 4-Me), 2.67 (2H, m, 3- and 4-H), 3.91 (6H, 3'- and 3"-OMe), 5.12 (2H, d, *J* = 6.6 Hz, 2- and 5-H), 5.59 (2H, s, 4'- and 4"-OH), 6.90-6.99 (6H, m, 2'-, 5'-, 6'-, 2"-, 5"-and 6"-H); ¹³C-NMR: ppm (125 MHz, CDCl₃): δ 11.7 (3- and 4-Me), 41.5 (C-3 and C-4), 55.8 (2×OMe), 82.7 (C-2 and C-5), 109.0 (C-2' and C-2"), 113.9 (C-5' and C-5"), 119.3 (C-6' and C-6"), 132.5 (C-1' and C-1"), 144.3 (C-4'and C-4"), 146.2 (C-3' and C-3").

### 3-2. Saucernetin (Compound 2)

Colorless oil; ¹H-NMR: ppm (500 MHz, CDCl₃): δ 0.63 (3H, d, *J* = 6.5 Hz, 4-Me), 1.01 (3H, d, *J* = 6.5 Hz, 3-Me), 2.33 (2H, m, 3- and 4-H), 3.90-3.92 (6H, 3'-and 3"-OMe), 4.65 (1H, d, *J* = 9.5 Hz, 2-H), 5.46 (1H, d, *J* = 4.5 Hz, 5-H), 5.58 (2H, s, 4'- and 4"-OH), 6.77-6.99 (6H, m, 2'-, 5'-, 6'-, 2"-, 5"- and 6"-H); ¹³C-NMR: ppm (125 MHz, CDCl₃): δ 9.42 (3-Me), 11.83 (4-Me), 43.4 (C-3), 47.6 (C-4), 55.8 (2×OMe), 84.8 (C-2), 85.7 (C-5), 108.3 (C-2'), 108.7 (C-2"), 113.9 (C-5'), 114.0 (C-5"), 118.8 (C-6'), 119.3 (C-6"), 132.6 (C-1'), 135.0 (C-1"), 144.3 (C-4'), 145.0 (C-4"), 146.3 (C-3"), 146.6 (C-3').

### 3-3. Verrucosin (Compound 3)

Colorless oil; ¹H-NMR: ppm (500 MHz, CDC1₃): δ 0.67 (3H, d, *J* = 6.5 Hz, 4-Me), 1.06 (3H, d, *J* = 6.5 Hz, 3-Me), 1.79 (1H, m, 3-H), 2.25 (1H, m, 4-H), 3.87-3.89 (6H, 3'- and 3"-OMe), 4.36 (1H, d, *J* = 9.5 Hz, 2-H), 5.10 (1H, d, *J* = 9.0 Hz, 5-H), 6.80-6.96 (6H, m, 2'-, 5'-, 6'-, 2"-, 5"- and 6"-H); ¹³C-NMR: ppm (125 MHz, CDCl₃): δ 14.8 (3-Me), 15.2 (4-Me), 45.9 (C-4), 46.8 (C-3), 56.4 (2×OMe), 84.6 (C-2), 89.0 (C-5), 111.6 (C-2'), 111.9 (C-2"), 115.7 (C-5'), 116.1 (C-5"), 120.7 (C-6'), 120.9 (C-6"), 133.0 (C-1'), 133.8 (C-1"), 147.5 (C-4'), 146.8 (C-4"), 148.6 (C-3"), 149.0 (C-3').

### 3-4. Nectandrin B (Compound 4)

Colorless oil; ¹H-NMR: ppm (600 MHz, CDCl₃): δ 1.05 (6H, d, *J* = 6.0 Hz, 3-and 4-Me), 2.35 (2H, m, 3- and 4-H), 3.85 (6H, 3'- and 3"-OMe), 4.53 (2H, d, *J* = 5.4 Hz, 2- and 5-H), 5.74 (2H, brs, 4'- and 4"-OH), 6.91 (2H, d, *J* = 7.8 Hz, 5'- and 5"-H), 6.93 (2H, dd, *J* = 1.8, 7.8 Hz, 6'- and 6"-H), 6.99 (2H, d, *J* = 1.8 Hz, 2'- and 2"-H); ¹³C-NMR: ppm (200 MHz, CDCl₃): δ 133.9 (C-1' and C-1"), 114.1 (C-2' and C-2"), 146.4 (C-3' and C-3"), 144.9 (C-4' and C-4"), 109.2 (C-5' and C-5"), 119.1 (C-6' and C-6"), 87.2 (C-2 and C-5), 44.1 (C-3 and C-4), 12.7 (3-Me and 4-Me), 55.7 (-OMe×2).

### 3-5. Nectandrin A (Compound 5)

White crystal; ¹H-NMR: ppm (500 MHz, CDCL₃) δ. 1.00 (3H, d, *J* = 3.5 Hz, 4-Me), 1.02 (3H, d, *J* = 3.5 Hz, 3-Me), 2.27 (2H, m, 3- and 4-H), 3.80-3.86 (9H, s, 3×OMe), 4.43 (1H, d, *J* = 7.5 Hz, 2-H), 4.44 (1H, d, *J* = 7.5 Hz, 5-H), 6.81-7.09 (6H, m, Ar-H); ¹³C-NMR: ppm (125 MHz, CDCL₃) δ. 13.1 (3-Me), 13.2 (4-Me), 45.6 (C-4), 45.5 (C-3), 56.1-56.3 (3×OMe), 88.0 (C-5), 88.2 (C-2), 110.9 (C-2'), 111.3 (C-2"), 112.7 (C-5'), 115.5 (C-5"), 119.5 (C-6'), 120.0 (C-6"), 135.1 (C-1'), 136.4 (C-1"), 146.9 (C-4'), 148.3 (C-4"), 149.9 (C-3"), 150.4 (C-3').

### 3-6. Fragransin C-1 (Compound 6)

Colorless oil; ¹H-NMR: ppm (500 MHz, CDCl₃): δ 1.04 (3H, d, *J* = 6.6 Hz, 3-Me), 1.06 (3H, d, *J* = 7.2 Hz, 4-H), 2.32 (1H, m, 3-H), 2.34 (1H, m, 4-H), 3.88 (9H, 3'-, 5'- and 3"-OMe), 4.50 (1H, d, *J* = 7.2 Hz, 2-H), 4.52 (1H, d, *J* = 7.2 Hz, 5-H), 5.47-5.58 (2H, br, s, 4'- and 4"-OH), 6.67 (2H, br, s, 2'- and 6'-H), 6.91-6.97 (3H, m, 2"-, 5"- and 6"-H), ¹³C-NMR: ppm (125 MHz, CDCl₃): δ 12.9(3-Me), 13.1 (4-Me), 44.0 (C-3), 44.5 (C-4), 56.3-55.8 (3×OMe), 87.2 (C-2), 87.5 (C-5), 103.1 (C-2' and C-6'), 102.9 (C-2"), 114.1 (C-5"), 119.3 (C-6"), 134.0 (C-1'), 133.5 (C-1"), 145.1 (C-4'), 146.4 (C-4"), 146.9 (C-3', C-5' and C-3").

### <Test Example 1: Measurement of AMPK-activating activity of lignan compounds 1-6>

The activating activity of Compounds 1-6 isolated and purified from the nutmeg extract in Example 3 was measured using the C2C12 myoblast cell line. C2C12 cells were seeded on a 6-well plate and cultured using DMEM medium containing 10% bovine serum. Then, the medium was replaced with DMEM containing 1% bovine serum in order to induce differentiation. The differentiated cells were maintained in serum-free DMEM for 16 hours and cultured for 2 hours after treating with the sample. Then, the cells were treated with SDS sample buffer and ultrasonically lysed. The cell lysate was subjected to 10% SDS-PAGE electrophoresis and proteins were fixed onto PVDF transfer membrane using a semi-dry transfer system. The membrane was reacted with 5% skim milk for 1 hour at room temperature and western blotting was carried out using AMPK and phosphorylated AMPK (phospho-AMPK, Thr172) antibodies. Also, since the increased AMPK activity during energy metabolism is known to increase the phosphorylation of ACCs (acetyl-CoA carboxylases 1 and 2), phosphorylated ACCs (phospho-ACCs) were observed for the cell lysate.

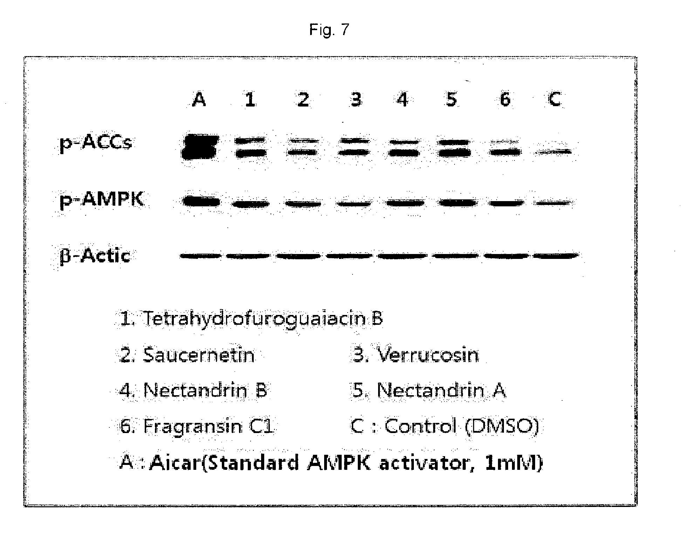

FIG. 7 shows the AMPK-activating activity of the lignan compounds 1-6 isolated from the nutmeg extract. As seen from FIG. 7, the lignan compounds 1-6 isolated from the nutmeg extract showed high level of AMPK and ACC activation. Since nectandrin B exhibited the highest content among the 2,5-bis-aryl-3,4-dimethyltetrahydrofuran lignan compounds as well as high AMPK activation as seen from FIG. 7, nectandrin B was used in the following experiments.

### <Test Example 2: Anti-obesity effect in diet-induced obese (DIO) model>

### [Step 1] Preparation of sample

Nectandrin B and macelignan were orally administered at a concentration of 100 mg/kg and 30% ethanol extract of nutmeg at 100 mg/kg every day. For the oral administration, the substances were dissolved in a 5% aqueous solution of methyl cellulose (Sigma Co., USA).

### [Step 2] Test groups and body weight loss effect

7-week-old male C57BL/6J mice were prepared for test, with 10 heads per group. After an accommodation period of 1 week, the mice were kept in individual cages and maintained with 12/12-hr light-dark cycles (lighting from 7 am to 5 pm). The test groups were: 1) high-fat diet group, 2) high-fat diet + nectandrin B 100 mg/kg/day group, 3) high-fat diet + macelignan 100 mg/kg/day group and 4) high-fat diet + 30% nutmeg extract 100 mg/kg/day group. The corresponding substance was orally administered once a day at regular hours (10 am) for 8 weeks. Body weight was measured once a week (11 am). After 8 weeks of administration, the body weight of the test groups and the control group was analyzed. Table 3 shows body weight change after the oral administration of the substances.

**Table 3**

| | Body weight change (g) after oral administration | | | | | | |
|---|---|---|---|---|---|---|---|
| | 14 days | 21 days | 28 days | 35 days | 42 days | 49 days | 56 days |
| High-fat diet | 4.40 ± 0.63 | 6.68 ± 0.70 | 8.04 ± 0.88 | 10.13 ± 0.87 | 11.49 ± 0.88 | 12.50 ± 0.87 | 13.40 ± 0.92 |
| Nectandrin B + high-fat diet | 2.73 ± 0.49 | 3.68 ± 0.63 | 4.39 ± 0.60 | 6.61 ± 0.62 | 7.44 ± 0.67 | 8.51 ± 0.80 | 8.89 ± 0.82 |
| Macelignan + high-fat diet | 4.00 ± 0.38 | 5.31 ± 0.54 | 6.52 ± 0.59 | 8.91 ± 0.72 | 9.70 ± 0.85 | 10.63 ± 0.84 | 11.48 ± 0.89 |
| Nutmeg 30% ethanol extract + high-fat diet | 3.22 ± 0.28 | 4.33 ± 0.39 | 5.20 ± 0.49 | 7.13 ± 0.48 | 8.39 ± 0.39 | 9.12 ± 0.51 | 9.55 ± 0.50 |

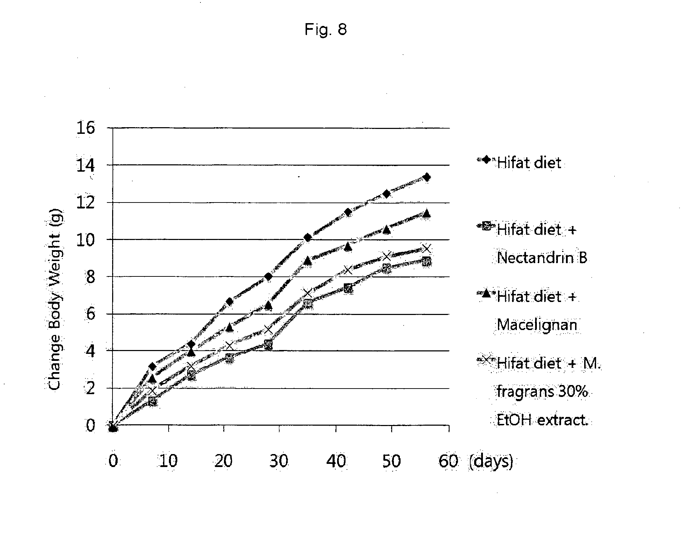

After 56 days of administration, the nutmeg extract + high-fat diet group and the nectandrin B + high-fat diet group showed less body weight increase as compared to the high-fat diet group and the macelignan + high-fat diet group, as seen from Table 3 and FIG. 8. The high-fat diet group showed a body weight increase of 13.40 g, whereas that of the nectandrin B group was 8.89 g. The suppression of body weight increase when compared with the high-fat diet group was statistically significant within 95% confidence level. In contrast, the macelignan group showed a body weight increase of 11.48 g, with statistically insignificant suppression of body weight increase as compared to the high-fat diet group. The 30% nutmeg extract group showed a body weight increase of 9.55 g, with statistically significant suppression of body weight increase within 95% confidence level when compared with the high-fat diet group (see Table 3).

### <Test Example 4: Toxicity test>

### 4-1. Acute toxicity

The acute toxicity (within 24 hours) and mortality of the active substances extracted from nutmeg was determined after administering the substances in large quantities. 20 ICR mice were divided into a control group (10) and a test group (10). The control group mice were orally administered only with PEG 400/Tween 80/EtOH (8/1/1, v/v/v), and the test group mice were orally administered with the active fractions extracted from nutmeg in Test Example 3 at 50 times the administration dosage of 100 mg/kg (i.e., 5 g/kg). Table 4 shows the result of orally administering the nutmeg extract or nectandrin B at 5 g/kg.

**Table 4**

| Orally administered substances | Time after administration (hr) | | | | | | |
|---|---|---|---|---|---|---|---|
| | 3 | 6 | 9 | 12 | 15 | 18 | 24 |
| Control (30% ethanol aqueous solution) | Survived | Survived | Survived | Survived | Survived | Survived | Survived |
| Nutmeg 30% ethanol extract | Survived | Survived | Survived | Survived | Survived | Survived | Survived |
| Nectandrin B | Survived | Survived | Survived | Survived | Survived | Survived | Survived |

As seen from Table 4, all the mice of the control group and the nutmeg extract and nectandrin B test groups survived 24 hours after the administration.

### 4-2. Organ and tissue toxicity

Organ toxicity test was carried out on the C57BL/6J mice used to test the anti-obesity effect. After administering nectandrin B or the 30% ethanol extract to the test groups and only the solvent to the control group for 8 weeks, blood was taken and glutamate-pyruvate transferase (GPT) and blood urea nitrogen (BUN) levels were measured using Select E (Vital Scientific NV, the Netherlands) in order to investigate the effect on individual organs (tissues). Table 5 shows the GTP and BUN levels after 8 weeks of oral administration of nutmeg 30% ethanol extract or nectandrin B.

**Table 5**

| Orally administered substances | Levels after 8 weeks | |
|---|---|---|
| | GTP | BUN |
| Control (30% ethanol) | 1.9 mg/dL | 2.1 mg/dL |
| Nutmeg 30% ethanol extract | 2.3 mg/dL | 2.2 mg/dL |
| Nectandrin B | 2.1 mg/dL | 1.8 mg/dL |

As a result, no significant difference was observed between the control group and the test groups in the levels of GPT, which is known to be related with hepatotoxicity, and BUN, which is known to be related with renal toxicity (see Table 5). And, when tissue sections were prepared from the liver and kidney of the animals and histologically observed under an optical microscope, no special abnormality was observed.

### <Formulation Example 1: Pharmaceutical formulations>

### 1-1. Preparation of tablet

The nutmeg extract according to the present disclosure or a compound isolated therefrom (200 g) was mixed with lactose (175.9 g), potato starch (180 g) and colloidal silicic acid (32 g). After adding 10% gelatin solution thereto, the mixture was pulverized and passed through a 14-mesh sieve. After drying, followed by addition of potato starch (160 g), talc (50 g) and magnesium stearate (5 g), the resulting mixture was prepared into tablet.

### 2-2. Preparation of injection solution

The compound isolated according to the present disclosure (1 g), sodium chloride (0.6 g) and ascorbic acid (0.1 g) were dissolved in distilled water and the volume was made to 100 mL. The resulting solution was put in a bottle and sterilized by heating for 30 minutes at 20 °C.

### <Formulation Example 2: Food formulations>

### 2-1. Preparation of seasoning

Seasoning for health improvement was prepared by using 0.2-10 parts by weight of the nutmeg extract according to the present disclosure or a compound isolated therefrom.

### 2-2. Preparation of wheat food

0.1-5.0 parts by weight of the nutmeg extract according to the present disclosure or a compound isolated therefrom was added to wheat flour. Bread, cake, cookie, cracker and noodle for health improvement were prepared using the resulting mixture.

### 2-3. Preparation of soup and gravy

0.1-1.0 parts by weight of the nutmeg extract according to the present disclosure or a compound isolated therefrom was added to soup or gravy to prepare soup or gravy for processed meat products or noodles for health improvement.

### 2-4. Preparation of dairy products

0.1-1.0 part by weight of the nutmeg extract according to the present disclosure or a compound isolated therefrom was added to milk. Various dairy products including butter and ice cream were prepared using the milk.

### 2-5. Preparation of vegetable juice

The nutmeg extract according to the present disclosure or a compound isolated therefrom (0.5 g) was added to tomato or carrot juice (1,000 mL) to prepare vegetable juice for health improvement.

### 2-6. Preparation of fruit juice

The nutmeg extract according to the present disclosure or a compound isolated therefrom (0.1 g) was added to apple or grape juice (1,000 mL) to prepare vegetable juice for health improvement.

The nutmeg extract extracted using an aqueous solution of ethanol according to the present disclosure contains the toxic substance myristicin at low content and contain the AMPK-activating 2,5-bisaryltetrahydrofuran lignan compounds at high concentrations. Since the nutmeg extract extracted under the extraction condition according to the present disclosure and the 2,5-bisaryltetrahydrofuran lignan compounds isolated therefrom activate AMPK, prevention and treatment of obesity or metabolic syndrome can be expected therefrom.

Also, since the toxic substance existing in the nutmeg extract can be removed, the present disclosure is usefully applicable to medicines, cosmetics, foods, or the like.

## Claims

1. A nutmeg extract extracted from nutmeg with an aqueous solution of 10-30% ethanol,
wherein the nutmeg extract is produced by further removing myristicin from the nutmeg extract by adsorption using an ion-exchange resin such that the nutmeg extract comprises 0.1 wt% or less of the toxic substance myristicin and comprises at least one lignan compound selected from nectandrin B, nectandrin A, fragransin C1, verrucosin, saucernetin and tetrahydrofuroguaiacin.

2. A health functional food for use in improving obesity or metabolic syndrome comprising the nutmeg extract according to claim 1.

3. The health functional food for use in improving obesity or metabolic syndrome according to claim 2, wherein the health functional food is selected from a group consisting of drink, meat, sausage, bread, candy, snack, noodle, dairy products including ice cream, soup, beverage including sports drink, alcoholic beverage, and nutritional supplements including vitamin complex.

4. A pharmaceutical composition for use in preventing or treating obesity or metabolic syndrome, comprising the nutmeg extract according to claim 1 as active ingredient and comprising a pharmaceutically acceptable carrier or excipient.

5. The pharmaceutical composition for use in preventing or treating obesity or metabolic syndrome according to claim 4, wherein the composition enhances the activity of AMP-activated protein kinase (AMPK).

6. A method for producing a nutmeg extract
comprising extracting from nutmeg with an aqueous solution of 10-30% ethanol a nutmeg extract,
further comprising removing myristicin from the nutmeg extract by adsorption using an ion-exchange resin,
wherein the nutmeg extract comprises 0.1 wt % or less of the toxic substance myristicin and comprises at least one lignan compound selected from nectandrin B, nectandrin A, fragransin C1, verrucosin, saucernetin and tetrahydrofuroguaiacin.

## Patentansprüche

1. Muskatnussextrakt, aus Muskatnuss mit einer wässrigen Lösung von 10 - 30% Ethanol extrahiert,
wobei der Muskatnussextrakt erzeugt wird, indem außerdem Myristicin durch Adsorption unter Verwendung eines Ionenaustauscherharzes aus dem Muskatnussextrakt entfernt wird, derart dass der Muskatnussextrakt 0,1 Gewichtsprozent oder weniger der toxischen Substanz Myristicin enthält und mindestens ein Lignan enthält, ausgewählt unter Nectandrin B, Nectandrin A, Fragransin C1, Verrucosin, Saucernetin und Tetrahydrofuroguaiacin.

2. Gesundheitsförderndes Lebensmittel zum Gebrauch zur Besserung von Adipositas oder Stoffwechselsyndrom, den Muskatnussextrakt nach Patentanspruch 1 enthaltend.

3. Gesundheitsförderndes Lebensmittel zum Gebrauch zur Besserung von Adipositas oder Stoffwechselsyndrom nach Patentanspruch 2, in dem das gesundheitsfördernde Lebensmittel aus einer Gruppe gewählt wurde, bestehend aus Getränk, Fleisch, Wurst, Brot, Bonbon, Imbiss, Nudeln, Milchprodukte einschließlich Speiseeis, Suppe, Getränk einschließlich Sportgetränke, alkoholische Getränke und Nahrungsergänzungsmitteln einschließlich Vitaminprodukten.

4. Pharmazeutische Zubereitung zur Anwendung in der Vorbeugung gegen oder Behandlung von Adipositas oder Stoffwechselsyndrom, den Muskatnussextrakt nach Patentanspruch 1 als Wirkstoff und ein pharmazeutisch akzeptables Vehikel oder Hilfsstoff enthaltend.

5. Pharmazeutische Zubereitung zur Anwendung in der Vorbeugung gegen oder Behandlung von Adipositas oder Stoffwechselsyndrom nach Patentanspruch 4, wobei die Zubereitung die Aktivität von AMP-aktivierter Proteinkinase (AMPK) verstärkt.

6. Verfahren zur Herstellung eines Muskatnussextraktes,
die Extraktion eines Muskatnussextraktes aus Muskatnuss mit einer wässrigen Lösung von 10 - 30% Ethanol umfassend, außerdem das Entfernen von Myristicin aus dem Muskatnussextrakt durch Adsorption unter Verwendung eines Ionenaustauscherharzes umfassend,
wobei der Muskatnussextrakt 0,1 Gewichtsprozent oder weniger der toxischen Substanz Myristicin enthält und mindestens eine Lignanverbindung enthält, ausgewählt unter Nectandrin B, Nectandrin A, Fragransin C1, Verrucosin, Saucernetin und Tetrahydrofuroguaiacin enthält.

## Revendications

1. Extrait de noix de muscade extrait de noix de muscade avec une solution aqueuse de 10 à 30% d'éthanol, l'extrait de noix de muscade étant produit en enlevant de plus la myristicine de l'extrait de noix de muscade par adsorption en utilisant une résine d'échange d'ions de telle manière que l'extrait de noix de muscade comprend 0,1% en poids ou moins de la substance toxique myristicine et comprend au moins un composé de lignane sélectionné parmi la nectandrine B, la nectandrine A, la fragransine C1, la verrucosine, la saucernétine et la tétrahydrofuroguaiacine.

2. Aliment fonctionnel diététique pour utilisation pour améliorer l'obésité ou le syndrome métabolique comprenant l'extrait de noix de muscade selon la revendication 1.

3. Aliment fonctionnel diététique pour utilisation pour améliorer l'obésité ou le syndrome métabolique selon la revendication 2, l'aliment fonctionnel diététique étant sélectionné dans un groupe composé de boissons, viandes, saucisses, pain, bonbons, snack, pâtes, produits laitiers y compris crème glacée, soupe, boissons y compris boissons pour sportifs, boissons alcoolisées et suppléments nutritionnels y compris complexe de vitamines.

4. Composition pharmaceutique pour utilisation pour la prévention ou le traitement de l'obésité ou du syndrome métabolique comprenant l'extrait de noix de muscade selon la revendication 1 en tant qu'ingrédient actif et comprenant un porteur ou un excipient acceptable sur le plan pharmaceutique.

5. Composition pharmaceutique pour utilisation pour la prévention ou le traitement de l'obésité ou du syndrome métabolique selon la revendication 4, la composition améliorant la protéine kinase activée par l'AMP (AMPK).

6. Procédé pour produire un extrait de noix de muscade comprenant l'extraction de noix de muscade avec une solution aqueuse de 10 à 30% d'éthanol par extrait de noix de muscade, comprenant de plus l'enlèvement de la myristicine de l'extrait de noix de muscade par adsorption en utilisant une résine d'échange d'ions, dans lequel l'extrait de noix de muscade comprend 0,1% en poids ou moins de la substance toxique myristicine et comprend au moins un composé de lignane sélectionné parmi la nectandrine B, la nectandrine A, la fragransine C1, la verrucosine, la saucernétine et la tétrahydrofuroguaiacine.
